# EUROPEAN PATENT SPECIFICATION

(11) **EP 4 181 841 B1**
(45) Date of publication and mention of the grant of the patent: **22.10.2025**
(21) Application number: 21745910.6
(22) Date of filing: 15.07.2021
(51) Int. Cl.: A61F 5/453

(54) **DEVICE FOR A MALE URINARY INCONTINENCE DEVICE**
VORRICHTUNG FÜR EINE HARNINKONTINENZVORRICHTUNG FÜR MÄNNER
DISPOSITIF POUR DISPOSITIF D'INCONTINENCE URINAIRE MASCULINE

(30) Priority: 17.07.2020 DK PA202070495
(43) Date of publication of application: 24.05.2023
(73) Proprietor: Coloplast A/S, 3050 Humlebaek (DK)
(72) Inventor: BECKER, Kim, 3400 Hilleroed (DK); ZEUTHEN, Kristoffer, 2100 Koebenhavn Ø (DK); STAGSTRUP, Rune, 2660 Broendby Strand (DK); BOUGHERARA, Chaban, 1879 Frederiksberg C (DK); BORG, Marie Bay, 2700 Broenshoej (DK)
(86) International application number: PCT/DK2021/050241
(87) International publication number: WO 2022/012729

(56) References cited:
- WO-A1-2019/214787
- WO-A1-2019/214788
- US-A1- 2015 190 305
- US-A1- 2015 374 535

## Description

The invention relates to a male urinary incontinence device.

### Brief Description of the Drawing

The accompanying drawings are included to provide a further understanding of embodiments and are incorporated into and a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Figure 1 is an isometric view of one embodiment of a male urinary incontinence device.
Figure 2 is an isometric view of an embodiment of a cuff member of a male urinary incontinence device.
Figure 3 is a distal view of an embodiment of a male urinary incontinence device.

### Detailed Description

Embodiments, and features of the various exemplary embodiments described in this application, may be combined with each other ("mixed and matched"), unless specifically noted otherwise.

In the following Detailed Description reference is made to the accompanying drawings, which form a part hereof, and in which is shown by way of illustration specific embodiments in which the invention may be practiced. Because components of embodiments can be positioned in different orientations, the directional terminology is used for purposes of illustration and is in no way limiting. It is to be understood that other embodiments may be utilized, and structural or logical changes may be made without departing from the scope of the present invention. The following detailed description, therefore, is not to be taken in a limiting sense, and the scope of the present invention is defined by the appended claims.

Throughout this disclosure, the words "patient" or "user" are used to address the person to wear the male urinary incontinence device. However, in some cases "user" may also relate or refer to a health care professional (HCP), such as a surgeon or continence care nurse or others. In those cases, it will either be explicitly stated, or be implicit from the context that the "user" is not the "patient" himself.

In the following, whenever referring to a proximal side of a device or part of a device, the proximal side is the side closest to the user, when the device is fitted on a user and the distal side is the opposite side - the side furthest away from the user in use.

The axial direction is defined as the direction of the penis, when the device is worn by a user. The radial direction is defined as transverse to the axial direction that is transversely to the direction of the penis. Thus, the radial direction is substantially perpendicular to the penis of the user. The longitudinal direction is the direction from the proximal top portion to the distal bottom portion. The transverse direction is the direction perpendicular to the longitudinal direction, which corresponds to the direction across an intermediate portion located between the proximal top portion and the distal bottom portion.

The use of the phrase "substantially" as a qualifier of certain features or effects throughout this disclosure is intended to simply mean that any deviations are within tolerances that would normally be expected by the skilled person in the relevant field.

Many people suffer from light incontinence. Light incontinence may occur as a result of various types of incontinence problems. Stress incontinence means that you leak urine when you sneeze, cough, laugh, lift something, change position, or do something that puts stress or strain on your bladder. Urge incontinence is an urge to urinate that is so strong that you cannot make it to the toilet in time. It also happens when your bladder squeezes when it shouldn't. This can happen even when you have only a small amount of urine in your bladder. Overactive bladder is a kind of urge incontinence. Where the amount of leaked urine in stress incontinence may be quite small such as few drops, the amount of leaked urine in urge incontinence may be larger, such as up to 180 ml a day.

Male urinary incontinence aiding systems are known. Widely used systems known as external urinary catheters and urisheaths normally comprise a roll-on sheath or body portion for enclosing the shaft of the penis, and a tip portion that is provided with a comparatively short discharge tube, which via a tube is connected to a urine collection bag that is fastened to the leg of the user. The sheath portion is rolled-up in a number of successive windings to such an extent that adhesive on the inner surface of the sheath is accommodated in the windings. However, unrolling of a sheath correctly on a penis can be very challenging and it is essential that the sheath is fitted correctly to ensure a good sealing between the skin of the penis and the sheath. The known systems are especially intended and designed for persons suffering from medium to high urine incontinence, and the urine collection bags having a capacity of 500 ml are rather big and inconvenient to wear.

WO 2019/214788 discloses a male urinary incontinence device. device according to the preamble of claim 1.

There is a need for a lightweight and discreet male urinary incontinence device that may conveniently be sealingly arranged on the penis and having a smaller capacity of about 100-300 ml and be more agreeable for the user to wear in case of lighter incontinence. Incontinent users and health care professionals alike would welcome improvements in continence care devices to better meet such requirements.

Embodiments relate to a male urinary incontinence device comprising a top end portion, a bottom end portion and an intermediate portion for containing at least a part of a penis, the top end portion comprising an inlet through which the penis can be entered, the inlet comprising a cuff member comprising a membrane with a through-going aperture, and a collar provided around the aperture, the collar extending axially away from a first surface of the membrane, the membrane and the collar is configured to allow elastic expansion of the aperture to fittingly engage with the penis, wherein the cuff member comprises a material with a hardness of 5 to 45 in Shore A scale, or 45 to 88 in Shore 00 scale.

In embodiments, the collar is extending substantially perpendicular to and axially away from a first surface of the membrane. In embodiments, the collar defines an angle with the membrane of 45-120 degrees. The angle between the collar and the membrane may be defined by the anatomy of the body but easy manufacturing of the cuff member may also be considered while choosing the angle.

In embodiments, the device is provided with an outlet at the bottom end portion. Such outlet may be connected, optionally via a tube or hose, to urine collecting means. In embodiments, the collecting means comprises a collecting bag. The outlet may be recloseable or it may be provided with a single use seal to be broken before use. In embodiments, the pocket may be closed at the bottom end portion. In embodiments, the device comprises an absorbent element configured to absorb urine.

In embodiments, the bottom end portion and the intermediate portion together defines a pocket. A distal wall and a proximal wall define the sides of the pocket. In embodiments, the pocket may comprise an absorbent element. The absorbent element is configured to absorb urine leaking from the penis. In embodiments, the absorbent element may be an integrated part of the device. The absorbent element may be attached to the distal wall inside the pocket. This may be a suitable solution for a single use device.

In embodiments, the absorbent element is a discrete element that can be exchanged and replaced with a fresh element without changing the whole device. In embodiments, the absorbent element may be provided with a handle for easy exchange of the absorbent element. Absorbent materials suitable for an absorbent element may comprise any absorbent material that is capable of absorbing and retaining fluids such as urine.

In embodiments, at least the bottom end portion is provided with a liquid barrier layer. In embodiments, the pocket is provided with a liquid barrier layer. The liquid barrier layer may comprise water impermeable material. In embodiments, the pocket comprises a water impermeable film layer. In embodiments, the liquid barrier layer is in the form of a coating. In embodiments, the pocket is splash-proof. In embodiments, the device is provided with a cover layer, such as a textile or non-woven to provide a pleasant surface towards the skin of the user as well as facilitate a nice appearance. In embodiments, the cover layer is on the outside surface of the device.

In embodiments, at least a part of the inside surface of the device is provided with a skin-friendly surface, such as a non-woven. Such skin-friendly surface may provide comfort for the user as well as it may have moisture-transporting properties, securing a dry surface against the skin.

The cuff member provides a sufficient sealing against the shaft of the penis and enables easy application of the device. In embodiments, the cuff member is water impermeable. In embodiments, the cuff member is water impermeable but moisture permeable. In embodiments, the cuff member is splash proof.

In embodiments, the cuff member serves to provide a splash proof fit to the penis. The collar of the cuff member may seal against the shaft of the penis, with a pressure sufficiently high as to provide at least a splash proof sealing, but not so high that it may cause discomfort for the user.

In embodiments, the cuff member comprises a flexible material such as an elastomer. In embodiments, the cuff member comprises a silicone material. In embodiments, the cuff member, comprising the membrane and the collar, is made from the same material and in one piece. The material of the cuff member may be soft, flexible and elastic. In embodiments, the cuff member comprises a thermoplastic elastomer. The elasticity of the cuff member may allow that the same cuff member can be fitted to a wide range of penis sizes as the collar and the membrane are designed to allow expansion of the through-going aperture. By flexible/elastic is herein meant that the material is able to stretch to fit around penises of different sizes.

From a manufacturing point of view thermoplastic elastomers are often preferred for the cuff member as they can be formed by injection molding. In embodiments, thermoset materials, such as silicone, may also be suitable material for the cuff member.

In embodiments, the cuff member of the device can be made from a thermoset elastomer, such as natural rubber latex, nitrile rubber latex, chloroprene rubber latex, SBS rubber latex or other synthetic lattices or from silicone or polyurethane dispersions or emulsions.

The mechanical properties of the cuff member may be determined with regard to hardness, elongation at break and tension set. The properties of the cuff member are designed to provide a cuff member being elastic enough to fit in a splash-proof manner to a wide range of penis sizes, in a manner where pressure marks and discomfort for the user is avoided.

The cuff member should be capable of being stretched and extended to be placed around a penis, but without breaking. In embodiments the elongation at break for the cuff member is 500 to 1100% tested according to DIN 53504/ISO 37, with a slight modification; deviating from ISO 37 standard test piece S2 is tested with a traverse speed of 200 mm/min.

When the cuff member has been stretched, it should be capable of regaining at least some of its original dimension in order to provide tight fit to the penis. This is measured as tension set, which is the ability of an elastomer to regain its original dimension (within tolerances) after being subjected to constant elongational stress. In embodiments, the cuff member has a tension set being between 0 and 10%, tension set is tested according to DIN ISO 2285 Method A and a test time of 24h with a laboratory temperature of 23°C.

The hardness of the cuff member is a measure of the resistance to indentation or abrasion. The hardness of the cuff member is measured in Shore A scale to which corresponds a Shore 00 scale (VLRH, Very Low Rubber Hardness). In embodiments, the hardness for the cuff member is from Shore A 5 to 45, or 45 to 88 in Shore 00 scale. Tested according to DIN ISO 27588 (VLRH 6 mm).

The length of the collar is measured in axial direction from the membrane. The length of the collar is chosen to be long enough to provide at least a splash proof fit to the penis and short enough to be comfortable for the user. In embodiments, the collar has a length between 5-30 mm, such as 7-25 mm such as 8-20 mm or even between 10-18 mm.

In embodiments, the presence of the collar facilitates better sealing and pressure distribution by detaching the pressure from the pocket. Furthermore, the collar may decrease the risk of pressure marks occurring at the penis.

Some male urinary incontinence devices are designed to hold themselves in place when applied to a penis. However, in order to have this, a tight fit is necessary, and this may give rise to pressure marks and discomfort for the user. In embodiments, the cuff member is not necessarily able to hold the device in place by itself. The device may be secured to the body by the underwear/pants of the user, allowing the fit to the penis to be softer and thereby avoiding discomfort for the user.

In embodiments, the collar defines a tubular structure, being substantially perpendicular to the membrane. The tubular structure may be of substantially equal diameter over the entire length.

In embodiments, the collar may have a thickness of 0.2-0.6 mm, such as 0.3-0.5 mm such as about 0.4 mm. The thickness of the collar is measured in radial direction.

In embodiments, the collar has an overall equal thickness, without areas with higher or lower thickness.

In embodiments, the cuff member has an overall equal thickness. In embodiments, the outer edge portion of the membrane has an enlarged thickness. The enlarged thickness renders the edge portion more robust and the thicker material facilitates easier welding of the proximal wall to the membrane.

In embodiments, the cuff member is oriented with the collar facing the inside of the device. In embodiments, the cuff member is oriented with the collar facing the outside (facing the body of the user) of the device. The collar facing the body of the user may facilitate that the cuff member does not roll away as well as it provides sealing against the penis. In embodiments, the collar is invertible in the sense it can be switched between facing towards and facing away from the user.

In embodiments, the through-going aperture is substantially circular or oval. In embodiments, the diameter of the aperture, measured in radial direction, is 18-40 mm, such as 19-35 mm, such as 20-32 mm.

In embodiments, the membrane is a sheet-like elastic layer. In embodiments, the membrane has a thickness of 0.2-0.8 mm, such as 0.3-0.5 mm. In embodiments, the membrane is substantially circular or oval. The aperture may be located centrally in the membrane or it may have a decentral position. In embodiments, the membrane has a diameter (measured at the broadest point if not circular) of 50-100 mm, such as 60-90 mm, such as 70-85 mm. In embodiments, the diameter of the membrane is at least twice the diameter of the through-going aperture.

In embodiments, the membrane has an overall equal thickness, without areas with higher or lower thickness. In embodiments, an outer edge portion of the membrane, the portion that is attached to the proximal wall, has an enhanced thickness, being larger than the thickness of the rest of the membrane. In embodiments, the cuff member is smooth, without any ribs or other parts of enhanced thickness compare to the rest of the collar. In embodiments, the outer edge portion of the membrane, with enhanced thickness, has a width, measured in radial direction, of 5-10 mm, such as 8-12 mm.

In embodiments, the device comprises a proximal wall and a distal wall, sealed together along the periphery to form a closed pocket. In embodiments, the cuff member is located in the proximal wall. In embodiments, the proximal wall and the distal wall together defines a hollow body and the cuff member defines the top end portion. In embodiments, the membrane constitutes at least a part of the proximal wall of the device. In embodiments, the membrane constitutes the entire proximal wall of the device.

In embodiments, the distal wall may be provided with an openable door in the form of a slit, extending at least partly from one side edge to another side edge of the distal wall. The openable door facilitates accessing the inside of the device. The slit may be formed by having an upper section of the distal wall to be at least partly overlying a lower section of the distal wall. The overlapping portion of the distal wall may be provided with means for closing the slit. In embodiments such closing means can be adhesive or Velcro. The slit facilitates the user to insert his hand through the slit and pull the penis through the aperture of the cuff member. When the penis is arranged in the pocket of the device, the slit can be closed. In embodiments, the closing means are splash-proof or even waterproof.

### Detailed Description of the Drawing

Embodiments, and features of the various exemplary embodiments described in this application, may be combined with each other ("mixed and matched"), unless specifically noted otherwise.

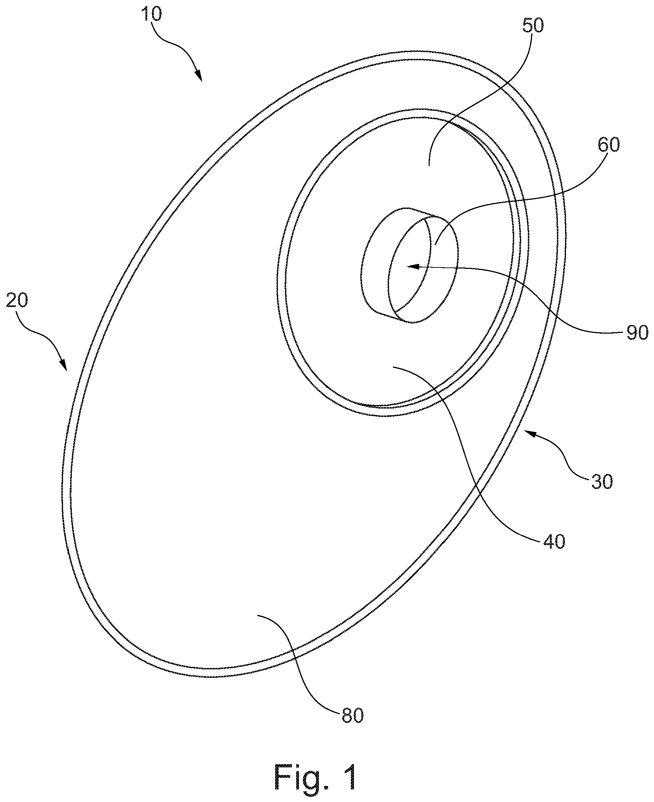

Figure 1 shows a device in perspective with a top end portion 10 a bottom end portion 20 and an intermediate portion 30 connecting the top end portion 10 and the bottom end portion 20. At the bottom end portion 20 may be provided an outlet (not shown) that may be used for leading urine from the device to a collecting bag, e.g. via a tube (not shown). The intermediate portion 30 is in the form of a hollow body extending from the top end portion 10 to the bottom end portion 20 and is configured to accommodating at least a length of a penis or at least the tip of a penis. The bottom end portion 20 and the intermediate portion 30 defines a pocket. At the top end portion 10 is provided a cuff member 40. The cuff member 40 comprises a membrane 50 with a through-going aperture 90 and a collar 60 provided around said aperture 90. The collar 60 is facing the outside of the device. The collar 60 is extending in axial direction away from the first surface of the membrane 50.

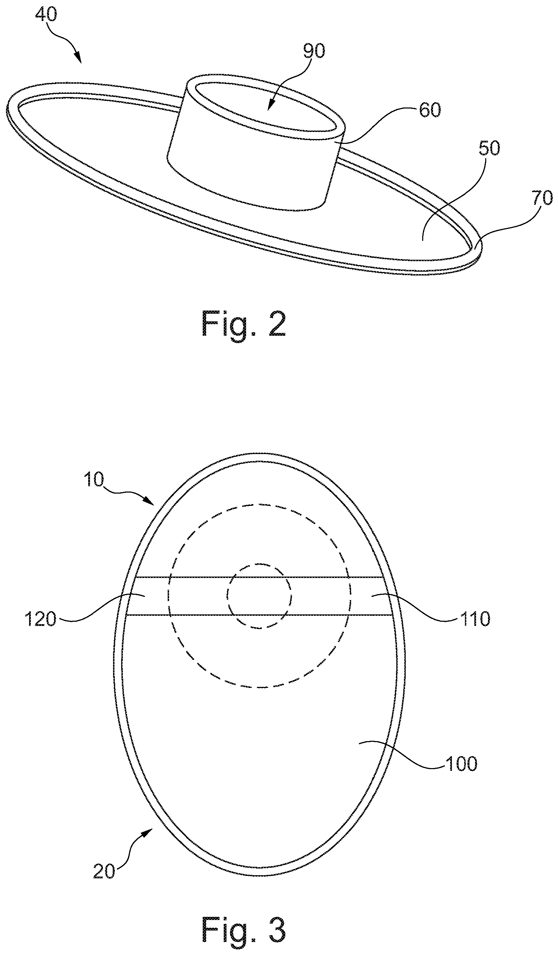

In Figure 2 is shown an embodiment of a cuff member 40 in perspective. The pocket is omitted from the drawing for clarity. The outer edge portion 70 of the membrane 50 has an enlarged thickness. The enlarged thickness renders the edge portion 70 more robust and the thicker material eases welding of the proximal wall 80 to the membrane 50.

In Figure 3 is shown an embodiment of a male urinary incontinence device seen from the distal side, facing the distal wall 100. Across the distal wall 100, extending at least partly from one side edge to another side edge, is provided a door in the form of a slit 110 for accessing the inside of the device. The slit 110 is formed by an upper overlapping section 120 of the distal wall overlying at least partly a lower section of the distal wall. The overlapping portion 120 of the distal wall may be provided with means for closing the slit 110, such as adhesive or Velcro. The slit facilitates the user to enter his hand through the slit 110 and pull the penis through the aperture 90 of the cuff member 40 when applying the device to the user. When the penis is arranged in the pocket of the device, the slit 110 can be closed with the overlapping section 120. The pocket of the device may be provided with absorbent means to control any flow of urine (not shown).

## Claims

1. A male urinary incontinence device comprising a top end portion (10), a bottom end portion (20) and an intermediate portion (30) for containing at least a part of a penis, the top end portion (10) comprising an inlet through which the penis can be entered, the inlet comprising a cuff member (40) comprising a membrane (50) with a through-going aperture (90), and a collar (60) provided around the aperture (90), the collar (60) extending axially away from a first surface of the membrane (50), the membrane (50) and the collar (60) is configured to allow elastic expansion of the aperture (90) to fittingly engage with the penis, **characterized in that** the cuff member (40) comprises a material with a hardness from 5 to 45 in Shore A scale, or from 45 to 88 in Shore 00 scale, the cuff member (40) has an elongation at break of 500 to 1100%, tested according to DIN 53504/ISO 37 with the modification that test piece S2 is tested with a traverse speed of 200 mm/min, and the tension set of the cuff member (40) is between 0 and 10%, tested according to DIN ISO 2285 Method A and a test time of 24h with a laboratory temperature of 23 °C.

2. The device according to any of the preceding claims, wherein the collar has a length of 5-30 mm.

3. The device according to any of the preceding claims, wherein the membrane has a diameter of 50-100 mm.

4. The device according to any of the preceding claims, wherein the collar has an overall equal thickness.

5. The device according to any of the preceding claims, wherein the membrane has an enlarged thickness along the outer edge portion.

6. The device according to claim 5, wherein the outer edge portion of the membrane with enhanced thickness has a width of 5-15 mm, measured in radial direction.

7. The device according to any of the preceding claims, wherein the collar is substantially perpendicular to the membrane.

8. The device according any of the preceding claims, wherein the diameter in a radial direction of the membrane is at least twice the diameter in a radial direction of the cuff member.

9. The device according to any of the preceding claims, wherein the collar and the membrane are made from the same material.

10. The device according to any of the preceding claims, wherein the cuff member comprises a thermoplastic elastomer.

## Patentansprüche

1. Vorrichtung für Harninkontinenz bei Männern, umfassend einen oberen Endabschnitt (10), einen unteren Endabschnitt (20) und einen Zwischenabschnitt (30) zum Enthalten wenigstens eines Teils eines Penis, wobei der obere Endabschnitt (10) einen Einlass umfasst, durch den der Penis eingeführt werden kann, wobei der Einlass ein Manschettenelement (40) umfasst, umfassend eine Membran (50) mit einer Durchgangsöffnung (90) und einen Kragen (60), der um die Öffnung (90) bereitgestellt ist, wobei der Kragen (60) axial von einer ersten Oberfläche der Membran (50) weg verläuft, wobei die Membran und der Kragen (60) dafür gestaltet sind, elastische Expansion der Öffnung (90) zu erlauben, um passend mit dem Penis in Eingriff zu kommen, **dadurch gekennzeichnet, dass** das Manschettenelement (40) ein Material mit einer Härte von 5 bis 45 auf der Shore-A-Skala oder von 45 bis 88 auf der Shore-00-Skala umfasst, das Manschettenelement (40) eine Bruchdehnung von 500 bis 1100 % aufweist, geprüft nach DIN 53504/ISO 37 mit der Modifikation, dass das Prüfstück S2 mit einer Quergeschwindigkeit von 200 mm/min geprüft wird, und der Zugverformungsrest des Manschettenelements (40) zwischen 0 und 10 % beträgt, geprüft nach DIN ISO 2285, Verfahren A, und mit einer Prüfdauer von 24 h bei einer Labortemperatur von 23 °C.

2. Vorrichtung nach einem der vorstehenden Ansprüche, wobei der Kragen eine Länge von 5-30 mm aufweist.

3. Vorrichtung nach einem der vorstehenden Ansprüche, wobei die Membran einen Durchmesser von 50-100 mm aufweist.

4. Vorrichtung nach einem der vorstehenden Ansprüche, wobei der Kragen eine allgemein gleiche Dicke aufweist.

5. Vorrichtung nach einem der vorstehenden Ansprüche, wobei die Membran eine erhöhte Dicke entlang des äußeren Randabschnitts aufweist.

6. Vorrichtung nach Anspruch 5, wobei der äußere Randabschnitt der Membran mit erhöhter Dicke eine Breite von 5-15 mm, gemessen in radialer Richtung, aufweist.

7. Vorrichtung nach einem der vorstehenden Ansprüche, wobei der Kragen im Wesentlichen senkrecht zu der Membran ist.

8. Vorrichtung nach einem der vorstehenden Ansprüche, wobei der Durchmesser in einer radialen Richtung der Membran wenigstens das Zweifache des Durchmessers des Manschettenelements in einer radialen Richtung ist.

9. Vorrichtung nach einem der vorstehenden Ansprüche, wobei der Kragen und die Membran aus dem gleichen Material bestehen.

10. Vorrichtung nach einem der vorstehenden Ansprüche, wobei das Manschettenelement ein thermoplastisches Elastomer umfasst.

## Revendications

1. Dispositif d'incontinence urinaire masculine comprenant une partie d'extrémité supérieure (10), une partie d'extrémité inférieure (20) et une partie intermédiaire (30) destinée à contenir au moins une partie d'un pénis, la partie d'extrémité supérieure (10) comprenant une entrée à travers laquelle le pénis peut pénétrer, l'entrée comprenant un élément de manchon (40) comprenant une membrane (50) avec une ouverture traversante (90), et un collier (60) prévu autour de l'ouverture (90), le collier (60) s'étendant axialement à l'opposé d'une première surface de la membrane (50), la membrane (50) et le collier (60) étant configurés pour permettre une expansion élastique de l'ouverture (90) pour venir en prise de manière ajustée avec le pénis, **caractérisé en ce que** l'élément de manchon (40) comprend un matériau ayant une dureté comprise entre 5 et 45 sur l'échelle Shore A, ou entre 45 et 88 sur l'échelle Shore 00, l'élément de manchon (40) ayant un allongement à la rupture de 500 à 1100 %, testé selon la norme DIN 53504/ISO 37 avec la modification que l'élément de test S2 est testé avec une vitesse transversale de 200 mm/min, et que la tension de l'élément de manchon (40) est comprise entre 0 et 10 %, testée selon la norme DIN ISO 2285, procédé A, et une durée d'essai de 24 h à une température de laboratoire de 23 °C.

2. Dispositif selon l'une quelconque des revendications précédentes, le collier ayant une longueur de 5 à 30 mm.

3. Dispositif selon l'une quelconque des revendications précédentes, la membrane ayant un diamètre de 50 à 100 mm.

4. Dispositif selon l'une quelconque des revendications précédentes, le collier ayant une épaisseur globale égale.

5. Dispositif selon l'une quelconque des revendications précédentes, la membrane ayant une épaisseur accrue le long de la partie de bord extérieur.

6. Dispositif selon la revendication 5, la partie de bord extérieur de la membrane à épaisseur accrue ayant une largeur de 5 à 15 mm, mesurée dans la direction radiale.

7. Dispositif selon l'une quelconque des revendications précédentes, le collier étant sensiblement perpendiculaire à la membrane.

8. Dispositif selon l'une quelconque des revendications précédentes, le diamètre dans une direction radiale de la membrane étant au moins deux fois le diamètre dans une direction radiale de l'élément de manchon.

9. Dispositif selon l'une quelconque des revendications précédentes, le collier et la membrane étant réalisés dans le même matériau.

10. Dispositif selon l'une quelconque des revendications précédentes, l'élément de manchon comprenant un élastomère thermoplastique.
